# EUROPEAN PATENT SPECIFICATION

(11) **EP 1 458 447 B1**
(45) Date of publication and mention of the grant of the patent: **09.09.2009**
(21) Application number: 02793018.9
(22) Date of filing: 12.12.2002
(51) Int. Cl.: A61P 31/18, A61K 31/365, A61K 31/635, A61K 31/425, A61K 31/435, A61K 31/47, A61K 31/495, A61K 45/06

(54) **COMBINATION OF CYTOCHOME P 450 DEPENDENT PROTEASE INHIBITORS**
KOMBINATION VON CYTOCHROM-P450-ABHÄNGIGEN PROTEASE-INHIBITOREN
COMBINAISON D'INHIBITEURS DE PROTEASE DEPENDANT DU CYTOCHROME P SB 450 /SB

(30) Priority: 12.12.2001 EP 01204841
(43) Date of publication of application: 22.09.2004
(73) Proprietor: Tibotec Pharmaceuticals Ltd., Eastgate Little Island Co Cork (IE)
(72) Inventor: VAN DER GEEST, Ronald, NL-4818 PD Breda (NL); STOFFELS, Paul, B-2320 Hoogstraten (BE); GROEN, Cornelis, NL-4713 RA Oudenbosch (NL); JOCHMANS, Dirk, Edward, Désiré, B-3020 Herent (BE)
(74) Representative: Daelemans, Frank F.R.
(86) International application number: PCT/EP2002/014277
(87) International publication number: WO 2003/049746

(56) References cited:
- WO-A-00/47551
- WO-A-95/10281
- WO-A-99/33815
- WO-A-99/67254
- HSU, ANN ET AL: "Ritonavir: clinical pharmacokinetics and interactions with other anti-HIV agents" CLINICAL PHARMACOKINETICS (1998), 35(4), 275-291, XP008004669
- TANAKA, E.: "Clinically important pharmacokinetic drug-drug interactions: role of cytochrome P450 enzymes" JOURNAL OF CLINICAL PHARMACY AND THERAPEUTICS (1998), 23(6), 403-416, XP000961726
- GHOSH A K ET AL: "Potent HIV protease inhibitors incorporating high-affinity P2-ligands and (R)-(hydroxyethylamino)sulfonamide isostere" BIOORGANIC & MEDICINAL CHEMISTRY LETTERS, OXFORD, GB, vol. 8, no. 6, 17 March 1998 (1998-03-17), pages 687-690, XP004136945 ISSN: 0960-894X

## Description

The present invention relates to improving the pharmacokinetics of hexahydrofuro[2,3-b]furanyl containing HIV protease inhibitors comprising administering to a human in need thereof a combination of a therapeutically effective amount of a hexahydrofuro[2,3-b]furanyl containing HIV protease inhibitor, and a therapeutically effective amount of a cytochrome P₄₅₀ inhibitor.

The virus causing the acquired immunodeficiency syndrome (AIDS) is known by different names, including T-lymphocyte virus III (HTLV-III) or lymphadenopathy-associated virus (LAV) or AIDS-related virus (ARV) or human immunodeficiency virus (HIV). Up until now, two distinct families have been identified, i.e. HIV-1 and HIV-2. Hereinafter, HIV will be used to generically denote these viruses.

One of the critical pathways in a retroviral life cycle is the processing of polyprotein precursors by retroviral protease. For instance, during the replication cycle of the HIV virus, gag and gag-pol gene transcription products are translated as proteins, which are subsequently processed by a virally encoded protease (or proteinase) to yield viral enzymes and structural proteins of the virus core. Most commonly, the gag precursor proteins are processed into the core proteins and the pol precursor proteins are processed into the viral enzymes, e.g., reverse transcriptase, integrase and retroviral protease. It has been shown that correct processing of the precursor proteins by the retroviral protease is necessary for the assembly of infectious virions. For example, it has been shown that frameshift mutations in the protease region of the pol gene of HIV prevent processing of the gag precursor protein. It has also been shown through site-directed mutagenesis of an aspartic acid residue in the HIV protease active site that processing of the gag precursor protein is prevented. Therefore, retroviral protease inhibition provides an attractive target for antiviral therapy. In particular for HIV treatment, the HIV protease is an attractive target.

Retroviral protease inhibition typically involves a transition-state mimetic whereby the retroviral protease is exposed to a mimetic compound which binds (typically in a reversible manner) to the enzyme in competition with the gag and gag-pol proteins to thereby inhibit specific processing of structural proteins and the release of retroviral protease itself. In this manner, retroviral replication proteases can be effectively inhibited.

HIV protease inhibitors (PIs) are commonly administered to AIDS patients in combination with other anti-HIV compounds such as, for instance nucleoside reverse transcriptase inhibitors (NRTIs), non-nucleoside reverse transcriptase inhibitors (NNRTIs) or other protease inhibitors.

Ghosh et al. (Bioorg. Med. Chem. Lett, 1998, 8, 687-690), WO 00/47551 and WO 99/33815 disclose certain HIV protease inhibitors comprising a hexahydrofuro[2,3-b]furanyl moiety.

Some antiretrovirals and, in particular, some HIV protease inhibitors are metabolized by cytochrome P₄₅₀, leading to sub-optimal pharmacokinetic profiles causing an undesired need for more frequent and higher doses. Thus, there is a high medical need for effective and safe anti-HIV treatment wherein the therapeutic compounds have good bioavailability, a favorable pharmacokinetic and metabolic profile, and have reduced side effects.

Several disclosures propose a combination of a protease inhibitor with at least one second compound for the improvement of the pharmacokinetic of said first PI. For instance, WO 00/25784 describes a method for improving the pharmacokinetics of tipranavir comprising a combination oftipranavir and ritonavir. US Pat. N° 6,180,634 discloses a synergistic composition comprising N-(2(R)-hydroxy-1(S)-indanyl)-2(R)-phenylmethyl-4-(S)-hydroxy-5-(1-(4-(2-benzo[b]furanylmethyl)-2(S)-N'-(t-butylcarboxamido)-piperazinyl))-pentaneamide and one or more antiretroviral agents such as indinavir. WO 97/01349 describes a method for improving the pharmacokinetics of a drug which is metabolized by cytochrome P₄₅₀ monooxygenase wherein said method comprises administering to a patient a composition comprising a combination of said drug with ritonavir. WO 95/10281 describes a combination of a selected protease inhibitor, L-735,524 in combination with either cimetidine or ketoconazole. Sadler et al. (AIDS, 2001, 15(8), 1009-1018) evaluated the pharmacokinetics and safety of amprenavir and ritonavir following multiple-dose, co-administration to healthy volunteers. Tanaka et al. (J. Clin. Pharmacy Therap., 1998, 23, 403-416) describe some HIV protease drugs whose metabolism may be dependent on isoforms of cytochrome P₄₅₀. Hsu et al. (Clin Pharmacokinet. 1998, 35, 275-291) describes the pharmacokinetics of Ritonavir, including the impact on cytochrome P₄₅₀ isoenzymes.

WO 99/67254 describes multi-drug resistant retroviral protease inhibitors.

It is an object of the present invention to provide improved combinations of hexahydrofuro[2,3-b]furanyl containing HIV protease inhibitors with cytochrome P₄₅₀ inhibitors. It is another object to provide a combination of hexahydrofuro[2,3-b]furanyl containing HIV protease inhibitors wherein a further synergistic effect of said inhibitors is observed upon administration of said composition to a patient in need thereof.

It has been found that the combination of (a) HIV protease inhibitors of formula **(4)** or a pharmaceutically acceptable salt thereof and (b) ritanovir or a pharmaceutically acceptable salt thereof, had a dose-reducing effect on the therapeutically effective dose of the HIV protease inhibitor of formula **(4).**

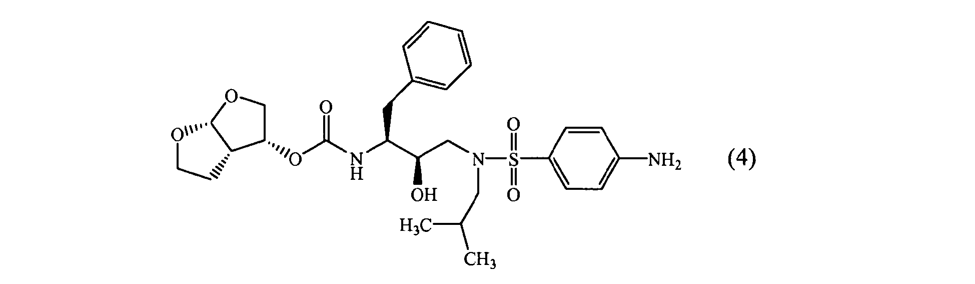

The present invention also relates to the use of said combination as a medicament for the treatment, the prevention or for combating retroviral infection. The present invention further relates to the use of said combination in the manufacture of a medicament for the treatment, prevention or for combating retroviral infection and in a method of treatment for retroviral infection. The present invention also relates to the use of said combination in high-throughput target-analyte assays such as, for example, phenotypic resistance monitoring assays.

As used herein, the term "composition" is intended to encompass a product comprising the specified ingredients, as well as any product which results, directly or indirectly, from combination of the specified ingredients.

Whenever the term "substituted" is used in defining the HIV protease inhibitor of formula **(4),** it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture, and formulation into a therapeutic agent.

In general, combinations of two compounds can act synergistically, in an additive way or antagonistically. Synergy between the two inhibitors would mean a more potent combination therapy, without increasing undesired side effects. For the current invention, this was assessed in an experimental setting where the potency of different ratios of the two HIV-protease inhibitors is measured. Results were plotted in an isobologram graph according to the method described by Chou and Talalay (Adv. Enzyme Regul. 22: 27-55, 1984) Antagonism on the contrary would preclude the combination and restrict the area of use. The effects of a combination of a compound of formula **(4)** in combination with each of the currently approved HIV protease inhibitors are described in the examples below (see example 3). The compound of formula **(4)** in combination with currently approved HIV protease inhibitors exhibits no antagonism. At all molar ratios the compound of formula **(4)** shows synergy with amprenavir, nelfinavir and ritonavir and it shows additive inhibition with indinavir and saquinavir.

The present invention relates to a combination comprising (a) an HIV protease inhibitor of formula **(4)** or a pharmaceutically acceptable salt thereof and (b) ritonavir or a pharmaceutically acceptable salt thereof. Said HIV protease inhibitor of formula **(4)** is carbamic acid [(1S,2R)-3-[[(4-aminophenyl)sulfonyl](2-methylpropyl)amino]-2-hydroxy-1-(phenylmethyl)propyl]-(3R, 3aS, 6aR)-hexahydrofuro[2,3-b]furan-3-yl ester.

Ritonavir is an inhibitor of P₄₅₀ 3A4 cytochrome. Cytochrome P₄₅₀ (CYP) 3A4 oxidizes a broad spectrum of drugs by a number of metabolic processes. When ritonavir is given in combination with an HIV protease inhibitor of formula **(4),** it increases the trough concentrations (Cₘᵢₙ) of such HIV protease inhibitor of formula **(4)** allowing reduction of the dose and dosing frequency.

Whenever used hereinafter, the term "HIV protease inhibitors of formula **(4)"** or similar term is meant to include the compounds of general formula **(4),** and their salts.

The HIV protease inhibitors of formula **(4)** according to the invention may also exist in their tautomeric forms. Such forms, although not explicitly indicated in the compounds described herein, are intended to be included within the scope of the present invention.

The term stereochemically isomeric forms of the compounds of general formula **(4)** defines all possible compounds made up of the same atoms bonded by the same sequence of bonds but having different three-dimensional structures which are not interchangeable, which the compounds of the present invention may possess. Unless otherwise mentioned or indicated, the chemical designation of a compound herein encompasses the mixture of all possible stereochemically isomeric forms which said compound may possess. Said mixture may contain all diastereomers and/or enantiomers of the basic molecular structure of said compound. All stereochemically isomeric forms of the components of a composition according to the invention either in pure form or in admixture with each other are intended to be embraced within the scope of the present invention.

For therapeutic use, the salts of the components comprised in a combination according to the invention, are those wherein the counterion is pharmaceutically or physiologically acceptable.

The pharmaceutically acceptable salts of the components comprised in the combinations of the present invention (in the form of water-, oil-soluble, or dispersible products) include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, phosphate, propionate, succinate, sulphate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such a sarginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl, and diamyl sulfates; long chain halides such as decyl, lauryl, myristyl and stearyl chlorides, bromides and iodides; aralkyl halides like benzyl and phenethyl-bromides and others. Other pharmaceutically acceptable salts include the sulfate salt ethanolate and sulfate salts.

The pharmaceutically acceptable salts of the components of the present combinations include the combination wherein one of the individual components is in the form of a pharmaceutically acceptable salt, the combination wherein all of the individual components are in the form of pharmaceutically acceptable salts, the combination wherein one or more of the individual components is in the form of a pharmaceutically acceptable salt while other of the components are used as the free base, or a pharmaceutically acceptable salt of the combined components (i.e., a salt of the combination). The pharmaceutically acceptable esters of the HIV protease inhibitors of formula **(1)** according to the invention refer to non-toxic esters, preferably the alkyl esters such as methyl, ethyl, propyl, isopropyl, butyl, isobutyl, or pentyl esters, of which the methyl ester is preferred. However, other esters such as phenyl-alkyl may be employed if desired.

Furthermore, the present invention relates to a pharmaceutical composition comprising a therapeutic amount of a combination according to the invention and a pharmaceutically acceptable excipient. More in particular, the present invention relates to a pharmaceutical composition comprising (a) a therapeutically effective amount of an HIV protease inhibitor of formula **(4)** and (b) a therapeutically effective amount of an inhibitor of cytochrome P₄₅₀, i.e. ritonavir and (c) a pharmaceutically acceptable excipient.

The pharmaceutical composition can be prepared in a manner known *per se* to one of skill in the art. For this purpose, at least one of an HIV protease inhibitor of formula **(4)** or any subgroup thereof, and ritonavir or a pharmaceutically acceptable salt thereof, together with one or more solid or liquid pharmaceutical excipients and, if desired, in combination with other pharmaceutical active compounds, are brought into a suitable administration form or dosage form which can then be used as a pharmaceutical in human medicine or veterinary medicine.

The term "therapeutically effective amount" as used herein means that amount of active compound or component or pharmaceutical agent that elicits the biological or medicinal response in a tissue, system, animal or human that is being sought, in the light of the present invention, by a researcher, veterinarian, medical doctor or other clinician, which includes alleviation of the symptoms of the disease being treated.
Since the instant invention refers to combinations comprising two or more agents, the "therapeutically effective amount" is that amount of the agents taken together so that the combined effect elicits the desired biological or medicinal response. For example, the therapeutically effective amount of a composition comprising (a) the compound of formula **(4)** and (b) ritonavir would be the amount of the compound of formula **(4)** and the amount of ritonavir that when taken together have a combined effect that is therapeutically effective.

According to the instant invention "a dose reducing effect on the therapeutically effective dose" means the effect of the inhibitor of cytochrome P₄₅₀ on the amount of a compound of formula **(4)** needed to elicit a therapeutic effect. It is an object of the instant invention that when the inhibitor of cytochrome P₄₅₀ is administered to a mammal in addition to a compound of formula **(4),** the inhibitor of cytochrome P₄₅₀ reduces the dose of the compound of formula **(4)** needed to elicit its therapeutic effect, when compared to the sole administration of said compound of formula **(4).**

Due to the favorable pharmacological properties of the combinations of the present invention, particularly its activity against retroviral protease enzymes, and more particularly its activity against multi-drug resistant HIV protease enzymes, said combination is useful in the treatment of individuals infected by HIV and for the prophylaxis of these individuals.

An advantage of the combination of the present invention is that the minimal concentrations of the compound of formula **(4)** are increased compared to the sole administration of said compound. If an HIV inhibitor is present in a concentration which does not prevent replication of the HIV virus, mutants of the HIV virus may emerge. It is known in the art that mutants of the HIV protease confer resistance to HIV protease inhibitors. Examples of such mutations comprise those mutations, independently selected from the list comprising mutations at amino acid positions 10, 20, 24, 30, 32, 33, 36, 46, 47, 48, 50, 53, 54, 63, 71, 73, 77, 82, 84, 88 or 90 in the HIV protease. The combination of the present invention may be useful to prevent or delay the onset of mutations in HIV protease, or if the HIV protease contains mutations at the initiation of therapy may prevent or delay the occurrence of additional mutations in the HIV protease.

It was now found that the combination of a compound of formula **(4)** together with ritonavir or a pharmaceutically acceptable salt thereof resulted in a reduced incidence of adverse effects. Thus, it was now found that the combination of a compound of formula **(4)** together with ritonavir or a pharmaceutically acceptable salt thereof has an improved safety and tolerability when compared to when the compound of formula **(1)** is administered alone.

The term "individual," as used herein refers to an animal, preferably a mammal, most preferably a human, who has been the object of treatment, observation or experiment.

Alternatively, the combinations of the present invention may also be formulated as a combined preparation for simultaneous, separate or sequential use in HIV therapy. In such a case, the compound of general formula **(4)** is formulated in a pharmaceutical composition containing other pharmaceutically acceptable excipients, and ritonavir or a pharmaceutically acceptable salt thereof is formulated separately in a pharmaceutical composition containing other pharmaceutically acceptable excipients. Conveniently, these two separate pharmaceutical compositions can be part of a kit for simultaneous, separate or sequential use.

Thus, the individual components of the combination of the present invention can be administered separately at different times during the course of therapy or concurrently in divided or single combination forms. The present invention is therefore to be understood as embracing all such regimes of simultaneous or alternating treatment and the term "administering" is to be interpreted accordingly.

The present invention further relates to the use of a combination according to the invention, in the treatment of individuals infected by a retrovirus and for the prophylaxis of these individuals. The prophylaxis treatment can be advantageous in cases where an individual has been subjected to a high risk of exposure to a virus, as can occur when individual has been in contact with an infected individual where there is a high risk of viral transmission. As an example, prophylactic administration of said composition would be advantageous in a situation where a health care worker has been exposed to blood from an HIV-infected individual, or in other situations where an individual engaged in high-risk activities that potentially expose that individual to the HIV virus.

In general, the combinations of the present invention may be useful in the treatment of warm-blooded animals infected with viruses whose existence is mediated by, or depends upon, a retroviral protease enzyme, in particular the HIV protease enzyme. Conditions which may be prevented or treated with the compositions of the present invention, especially conditions associated with HIV and other pathogenic retroviruses, include AIDS, AIDS-related complex (ARC), progressive generalized lymphadenopathy (PGL), as well as chronic CNS diseases caused by retroviruses, such as, for example HIV mediated dementia and multiple sclerosis.

The combinations of the present invention may therefore be used as medicaments against above-mentioned conditions. Said use as a medicine or method of treatment comprises the systemic administration to HIV-infected subjects of an amount effective to combat the conditions associated with HIV and other pathogenic retroviruses, especially HIV-1. Consequently, the combinations of the present invention can be used in the manufacture of a medicament useful for treating, preventing or combating infection or disease associated with retrovirus infection in a mammal, in particular for treating conditions associated with HIV and other pathogenic retroviruses, more in particular medicaments useful for treating patients infected with multi-drug resistant HIV virus.

The present invention further relates to the use of a combination according to the invention in the manufacture of a medicament for inhibiting a protease of a retrovirus in a mammal infected with said retrovirus. The present invention also relates to the use of a combination according to the invention in the manufacture of a medicament for inhibiting retroviral replication, in particular, when the retrovirus is a human immunodeficiency virus (HIV) and more in particular when the retrovirus is a multidrug-resistant retrovirus.

The present invention further encompasses a report comprising information obtained in any of the above described uses of a combination according to the invention.

Treating AIDS or preventing or treating infection by HIV is defined as including, but not limited to, treating a wide range of states of HIV infection: AIDS, ARC, both symptomatic and asymptomatic, and actual or potential exposure to HIV. The compositions of the present are also useful for treating progressive generalized lymphadenophaty, Kaposi's syndrome, thrombocytopenia purpurea, AIDS-related neurological conditions such as AIDS dementia complex, multiple sclerosis, tropical parapesis, and also anti-HIV antibody positive and HIV-positive conditions, including such conditions in asymptomatic patients. For example, the combinations of this invention are useful in treating infection by HIV after suspected past exposure to HIV by e.g., blood transfusion, exchange of body fluids, bites, accidental needle stick, or exposure to patient blood during surgery. The term prevention includes prophylaxis of HIV infection and prophylaxis of the evolution of HIV infection to AIDS.

For these purposes, the compositions comprising a combination of the present invention, whether co-formulated in a single formulation or formulated for simultaneous, separate or sequential use, may be administered orally (including suspensions, capsules, tablets, sachets, solutions, suspensions, emulsions), parenterally (including subcutaneous injections, intravenous, intramuscular, intrasternal injection or infusion techniques), by inhalation spray (including nasal sprays), or rectally (including suppositories), in dosage unit formulations containing conventional non-toxic pharmaceutically acceptable carriers, adjuvants and vehicles.

Another aspect of the present invention concerns a kit or container comprising a combination according to the invention combining an HIV protease inhibitor of formula **(4)** and ritonavir or a pharmaceutically acceptable salt thereof, in an amount effective for use as a standard or reagent in a test or assay for determining the ability of potential pharmaceuticals to inhibit HIV protease, HIV growth, or both. This aspect of the invention may find its use in pharmaceutical research programs.

The combinations of the present invention can be used in high-throughput target-analyte assays such as those for measuring the efficacy of said combination in HIV treatment.

The combinations of the present invention can be used in phenotypic resistance monitoring assays, such as known recombinant assays, in the clinical management of resistance developing diseases such as HIV. A particularly useful resistance monitoring system is a recombinant virus assay known as the Antivirogram^{™}. The Antivirogram^{™} is a highly automated, high throughput, second generation, recombinant assay that can measure susceptibility, especially viral susceptibility, to the compositions of the present invention. (Hertogs K, de Bethune MP, Miller V et al. Antimicrob Agents Chemother, 1998; 42(2):269-276).

In accordance with the present invention there is further provided a method for improving the pharmacokinetics of HIV protease inhibitor of formula **(4)** which is metabolized by cytochrome P₄₅₀ comprising administering to an individual in need of such treatment a therapeutically effective amount of a combination as described above comprising (a) said HIV protease inhibitor of formula **(4)** or a pharmaceutically acceptable salt thereof and (b) ritonavir or a pharmaceutically acceptable salt thereof.

The pharmacokinetics of an HIV protease inhibitor of formula (4) may be described using pharmacokinetic parameters known to the person skilled in the art. Examples of such parameters include: t_{1/2} (half life), Cₘᵢₙ (minimal concentration, trough concentration), Cₘₐₓ (maximal concentration), AUC (area under the curve), time to maximal concentration, steady state concentration (Cₛₛ).

The present invention further relates to treating HIV infection and AIDS comprising administering to a patient in need of such treatment a combination of the present invention comprising a therapeutically effective amount of each component of said combination.

In the present invention, the combination of HIV protease inhibitor of formula **(4)**, and ritonavir, can be administered concurrently in divided or single combination forms.

In another embodiment of the method of the invention, the administration may be performed with food (e.g., a high-fat meal) or without food. The term "with food" means the consumption of a meal either during or no more than about one hour before or after administration of a one or both components of the combination according to the invention.

For an oral administration form, the compositions of the present invention can be mixed with suitable additives, such as excipients, stabilizers or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose, or starch, in particular, corn starch. In this case, the preparation can be carried out both as dry and as moist granules. Suitable oily excipients or solvents are vegetable or animal oils, such as sunflower oil or cod liver oil. Suitable solvents for aqueous or alcoholic solutions are water, ethanol, sugar solutions, or mixtures thereof. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.

The oral administration of a combination comprising (a) an HIV protease inhibitor of formula **(4)** and (b) ritonavir, or a pharmaceutically acceptable salt of either or both, is suitably accomplished by uniformly and intimately blending together a suitable amount of each component in the form of a powder, optionally also including a finely divided solid carrier, and encapsulating the blend in, for example, a hard gelatin capsule. The solid carrier can include one or more substances which act as binders, lubricants, disintegrating agents, coloring agents, and the like. Suitable solid carriers include, for example, calcium phosphate, magnesium stearate, talc, sugars, lactose, dextrin, starch, gelatin, cellulose, polyvinylpyrrolidine, low melting waxes and ion exchange resins.

Oral administration of a composition comprising for example a combination of the compound of formula **(4)** and ritonavir in suitable proportions can also be accomplished by preparing capsules or tablets containing the desired amount of the compound of formula **(4)** only, optionally blended with a solid carrier as described above, and capsules containing the desired amount of ritonavir only. Compressed tablets containing the compound of formula **(4)** can be prepared by uniformly and intimately mixing the active ingredient with a solid carrier such as described above to provide a mixture having the necessary compression properties, and then compacting the mixture in a suitable machine to the shape and size desired. Molded tablets maybe made by molding in a suitable machine, a mixture of powdered the compound of formula **(4)** moistened with an inert liquid diluent. Oral administration can also be accomplished by preparing compressed or molded tablets containing the compound of formula **(4)** as just described, the tablets of suitable size for insertion into standard capsules (e.g., hard gelatin capsules), and then inserting the tablets into capsules containing a suitable amount of ritonavir powder.

When administered by nasal aerosol or inhalation, these compositions may be prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. Suitable pharmaceutical formulations for administration in the form of aerosols or sprays are, for example, solutions, suspensions or emulsions of the components of the compositions or their physiologically tolerable salts in a pharmaceutically acceptable solvent, such as ethanol or water, or a mixture of such solvents. If required, the formulation can also additionally contain other pharmaceutical auxiliaries such as surfactants, emulsifiers and stabilizers as well as a propellant. Such a preparation customarily contains the active compounds in a concentration from approximately 0.1 to 50%, in particular from approximately 0.3 to 3% by weight.

For subcutaneous or intravenous administration, the active components of the compositions, if desired with the substances customary therefore such as solubilizers, emulsifiers or further auxiliaries, are brought into solution, suspension, or emulsion. The components of the compositions can also be lyophilized and the lyophilizates obtained used, for example, for the production of injection or infusion preparations. Suitable solvents are, for example, water, physiological saline solution or alcohols, e.g. ethanol, propanol, glycerol, in addition also sugar solutions such as glucose or mannitol solutions, or alternatively mixtures of the various solvents mentioned. The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these formulations may be prepared by mixing the individual components of a composition according to the invention with a suitable non-irritating excipient, such as cocoa butter, synthetic glyceride esters or polyethylene glycols, which are solid at ordinary temperatures, but liquidify and/or dissolve in the rectal cavity to release the drug.

In order to enhance the solubility and/or the stability of the components of a pharmaceutical composition according to the invention, it can be advantageous to employ α-, β- or γ-cyclodextrins or their derivatives. In addition, co-solvents such as alcohols may improve the solubility and/or the stability of the components of the pharmaceutical compositions. In the preparation of aqueous compositions, addition salts of the components of said compositions are obviously more suitable due to their increased water solubility.

Appropriate cyclodextrins are α-, β- or γ-cyclodextrins (CDs) or ethers and mixed ethers thereof wherein one or more of the hydroxy groups of the anhydroglucose units of the cyclodextrin are substituted with alkyl, particularly methyl, ethyl or isopropyl, e.g. randomly methylated β-CD; hydroxyalkyl, particularly hydroxyethyl, hydroxypropyl or hydroxybutyl; carboxyalkyl, particularly carboxymethyl or carboxyethyl; alkylcarbonyl, particularly acetyl; alkyloxycarbonylalkyl or carboxyalkyloxyalkyl, particularly carboxymethoxypropyl or carboxyethoxypropyl; alkylcarbonyloxyalkyl, particularly 2-acetyloxypropyl. Especially noteworthy as complexants and/or solubilizers are β-CD, randomly methylated β-CD, 2,6-dimethyl-β-CD, 2-hydroxyethyl-β-CD, 2-hydroxyethyl-γ-CD, 2-hydroxypropyl-γ-CD and (2-carboxymethoxy)-propyl-β-CD, and in particular 2-hydroxypropyl-β-CD (2-HP-β-CD). The term mixed ether denotes cyclodextrin derivatives wherein at least two cyclodextrin hydroxy groups are etherified with different groups such as, for example, hydroxypropyl and hydroxyethyl. An interesting way of formulating the components of the compositions in combination with a cyclodextrin or a derivative thereof has been described in EP-A-721,331. Although the formulations described therein are with antifungal active ingredients, they are equally interesting for formulating the components of the compositions. Said formulations may also be rendered more palatable by adding pharmaceutically acceptable sweeteners and/or flavors.

More in particular, the combinations may be formulated in a pharmaceutical formulation comprising a therapeutically effective amount of particles consisting of a solid dispersion comprising the following components: (a) an HIV protease inhibitor of formula **(4)**, (b) ritonavir or a pharmaceutically acceptable salt thereof and (c) one or more pharmaceutically acceptable water-soluble polymers.

The term "a solid dispersion" defines a system in a solid state (as opposed to a liquid or gaseous state) comprising at least two components, wherein one component is dispersed more or less evenly throughout the other component or components. When said dispersion of the components is such that the system is chemically and physically uniform or homogenous throughout or consists of one phase as defined in thermodynamics, such a solid dispersion is referred to as "a solid solution". Solid solutions are preferred physical systems because the components therein are usually readily bioavailable to the organisms to which they are administered. The term "a solid dispersion" also comprises dispersions that are less homogenous throughout than solid solutions. Such dispersions are not chemically and physically uniform throughout or comprise more than one phase.

The water-soluble polymer in the particles is conveniently a polymer that has an apparent viscosity of 1 to 100 mPa.s when dissolved in a 2 % aqueous solution at 20°C solution. Preferred water-soluble polymers are hydroxypropyl methylcelluloses or HPMC. HPMC having a methoxy degree of substitution from about 0.8 to about 2.5 and a hydroxypropyl molar substitution from about 0.05 to about 3.0 are generally water soluble. Methoxy degree of substitution refers to the average number of methyl ether groups present per anhydroglucose unit of the cellulose molecule. Hydroxypropyl molar substitution refers to the average number of moles of propylene oxide which have reacted with each anhydroglucose unit of the cellulose molecule. The particles as defined hereinabove can be prepared by first preparing a solid dispersion of the components, and then optionally grinding or milling that dispersion. Various techniques exist for preparing solid dispersions including melt-extrusion, spray-drying and solution-evaporation, melt-extrusion being preferred.

It may further be convenient to formulate the components of the combination in the form of nanoparticles which have a surface modifier adsorbed on the surface thereof in an amount sufficient to maintain an effective average particle size of less than 1000 nm. Useful surface modifiers are believed to include those which physically adhere to the surface of the antiretroviral agent but do not chemically bind to the antiretroviral agent. Suitable surface modifiers can preferably be selected from known organic and inorganic pharmaceutical excipients. Such excipients include various polymers, low molecular weight oligomers, natural products and surfactants. Preferred surface modifiers include nonionic and anionic surfactants.

Yet another interesting way of formulating the components of the combination involves a pharmaceutical composition whereby the components are incorporated in hydrophilic polymers and applying this mixture as a coat film over many small beads, thus yielding a composition with good bioavailability which can conveniently be manufactured and which is suitable for preparing pharmaceutical dosage forms for oral administration. Said beads comprise (a) a central, rounded or spherical core, (b) a coating film of a hydrophilic polymer and an antiretroviral agent and (c) a seal-coating polymer layer. Materials suitable for use as cores in the beads are manifold, provided that said materials are pharmaceutically acceptable and have appropriate dimensions and firmness. Examples of such materials are polymers, inorganic substances, organic substances, and saccharides and derivatives thereof.

The combinations of this invention can be administered to humans in dosage ranges specific for each component comprised in said combinations. The components comprised in said combinations can be administered together or separately. HIV protease inhibitor of formula **(4)**, and ritonavir or a pharmaceutically acceptable salt thereof, may have dosage levels of the order of 0.02 to 5.0 grams-per-day.

When HIV protease inhibitor of formula **(4)** and ritonavir are administered in combination, the weight ratio of HIV protease inhibitor of formula **(4)** to ritonavir is suitably in the range of from about 40:1 to about 1:15, or from about 30:1 to about 1: 15, or from about 15: 1 to about 1: 15, typically from about 10: 1 to about 1:10, and more typically from about 8:1 to about 1:8. Also useful are weight ratios of HIV protease inhibitor of formula **(4)** to ritonavir ranging from about 6:1 to about 1:6, or from about 4:1 to about 1:4, or from about 3:1 to about 1:3, or from about 2:1 to about 1:2, or from about 1.5:1 to about 1:1.5. In one aspect, the amount by weight of HIV protease inhibitor of formula **(4)** is equal to or greater than that of ritonavir, wherein the weight ratio of HIV protease inhibitor of formula **(4)** to ritonavir is suitably in the range of from about 1: 1 to about 15: 1, typically from about 1: 1 to about 10: 1, and more typically from about 1: 1 to about 8: 1. Also useful are weight ratios of HIV protease inhibitor of formula **(4)** to ritonavir ranging from about 1: 1 to about 6: 1, or from about 1: 1 to about 5: 1, or from about 1: 1 to about 4:1, or from about 3:2 to about 3:1, or from about 1:1 to about 2:1 1 or from about 1:1 to about 1.5:1.

According to one embodiment, the compound of formula **(4)** and ritonavir may be co-administered twice a day, preferably orally, wherein the amount of the compound of formula (4) per dose is from about 10 to about 2500 mg, and the amount of ritonavir per dose is from 10 to about 2500 mg. In another embodiment, the amounts per dose for twice daily co-administration are from about 50 to about 1500 mg of the compound of formula (4) and from about 50 to about 1500 mg of ritonavir. In still another embodiment, the amounts per dose for twice daily co-administration are from about 100 to about 1000 mg of the compound of formula **(4)** and from about 100 to about 800 mg of ritonavir. In yet another embodiment, the amounts per dose for twice daily co-administration are from about 150 to about 800 mg of the compound of formula **(4)** and from about 100 to about 600 mg of ritonavir. In yet another embodiment, the amounts per dose for twice daily co-administration are from about 200 to about 600 mg of the compound of formula **(4)** and from about 100 to about 400 mg of ritonavir. In yet another embodiment, the amounts per dose for twice daily co-administration are from about 200 to about 600 mg of the compound of formula **(4)** and from about 20 to about 300 mg of ritonavir. In yet another embodiment, the amounts per dose for twice daily co-administration are from about 100 to about 400 mg of the compound of formula **(4)** and from about 40 to about 100 mg of ritonavir.

Exemplary combinations of the compound of formula **(4)** (mg)/ritonavir (mg) for twice daily dosage include 50/100, 100/100, 150/100, 200/100, 250/100, 300/100, 350/100, 400/100, 450/100, 50/133, 100/133, 150/133, 200/133, 250/133, 300/133, 50/150, 100/150, 150/150, 200/150, 250/150, 50/200, 100/200, 150/200, 200/200, 250/200, 300/200, 50/300, 80/300, 150/300, 200/300, 250/300, 300/300, 200/600, 400/600, 600/600, 800/600, 1000/600, 200/666, 400/666, 600/666, 800/666, 1000/666, 1200/666, 200/800, 400/800, 600/800, 800/800, 1000/800, 1200/800, 200/1200, 400/1200, 600/1200, 800/1200, 1000/1200, and 1200/1200. Other exemplary combinations of the compound of formula **(4)** (mg)/ritonavir (mg) for twice daily dosage include 1200/400, 800/400, 600/400, 400/200, 600/200, 600/100, 500/100, 400/50, 300/50, and 200/50.

According to another embodiment, the compound of formula **(4)** and ritonavir may be co-administered once a day, preferably orally, wherein the amount of the compound of formula **(4)** per dose is from about 10 to about 2500 mg, and the amount of ritonavir per dose is from 10 to about 2500 mg. In another embodiment, the amounts per dose for single daily co-administration are from about 50 to about 1500 mg of the compound of formula **(4)** and from about 50 to about 1500 mg of ritonavir. In still another embodiment, the amounts per dose for single daily co-administration are from about 100 to about 1000 mg of the compound of formula **(4)** and from about 100 to about 800 mg of ritonavir. In yet another embodiment, the amounts per dose for single daily co-administration are from about 150 to about 800 mg of the compound of formula **(4)** and from about 100 to about 600 mg of ritonavir. In yet another embodiment, the amounts per dose for single daily co-administration are from about 200 to about 600 mg of the compound of formula **(4)** and from about 100 to about 400 mg of ritonavir. In yet another embodiment, the amounts per dose for single daily co-administration are from about 200 to about 600 mg of the compound of formula (4) and from about 20 to about 200 mg of ritonavir. In yet another embodiment, the amounts per dose for single daily co-administration are from about 100 to about 400 mg of the compound of formula (4) and from about 40 to about 100 mg of ritonavir.

Exemplary combinations of the compound of formula **(4)** (mg)/ritonavir (mg) for single daily dosage include 50/100, 100/100, 150/100, 200/100, 250/100, 300/100, 350/100, 400/100, 450/100, 50/133, 100/133, 150/133, 200/133, 250/133, 300/133, 50/150, 100/150, 150/150, 200/150, 250/150, 50/200, 100/200, 150/200, 200/200, 250/200, 300/200, 50/300, 80/300, 150/300, 200/300, 250/300, 300/300, 200/600, 400/600, 600/600, 800/600, 1000/600, 200/666, 400/666, 600/666, 800/666, 1000/666, 1200/666, 200/800, 400/800, 600/800, 800/800, 1000/800, 1200/800, 200/1200, 400/1200, 600/1200, 800/1200, 1000/1200, and 1200/1200. Other exemplary combinations of the compound of formula **(4)** (mg)/ritonavir (mg) for once daily dosage include 1200/400, 800/400, 600/400, 400/200, 600/200, 600/100, 500/100, 400/50, 300/50, 200/50.

It will be understood, however, that specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The following examples are meant to be illustrative of the present invention.

### Brief description of the figures

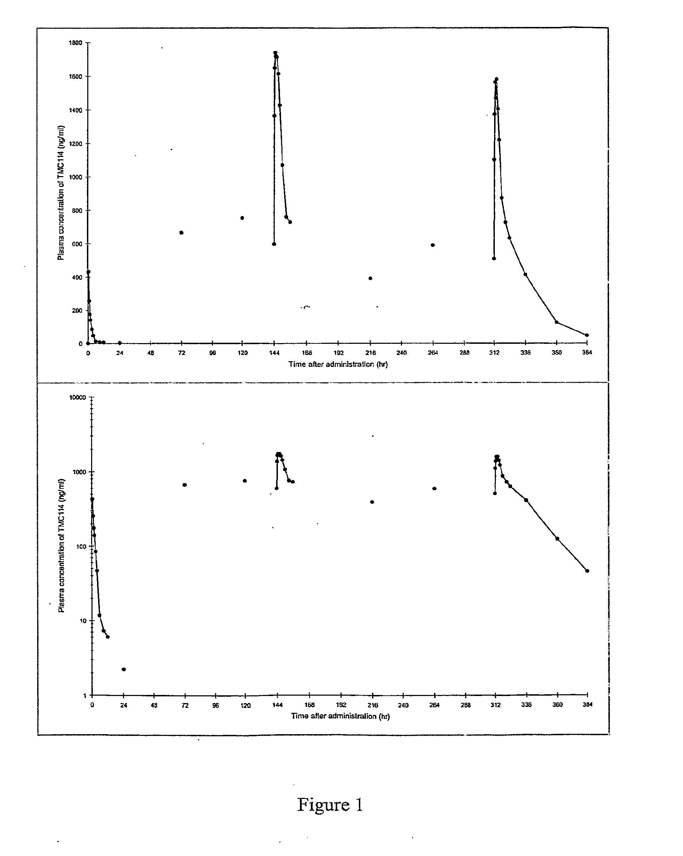

Figure 1: Represents a mean concentration-time profile from a clinical trial with a combination of the compound of formula **(4)** with ritonavir, wherein the panel was subjected to oral administration of 200 mg the compound of formula **(4)** o.d. (once daily) on day 1-14 and 100 mg ritonavir o.d. on day 2-16. The bottom figure is on a logarithmic scale.

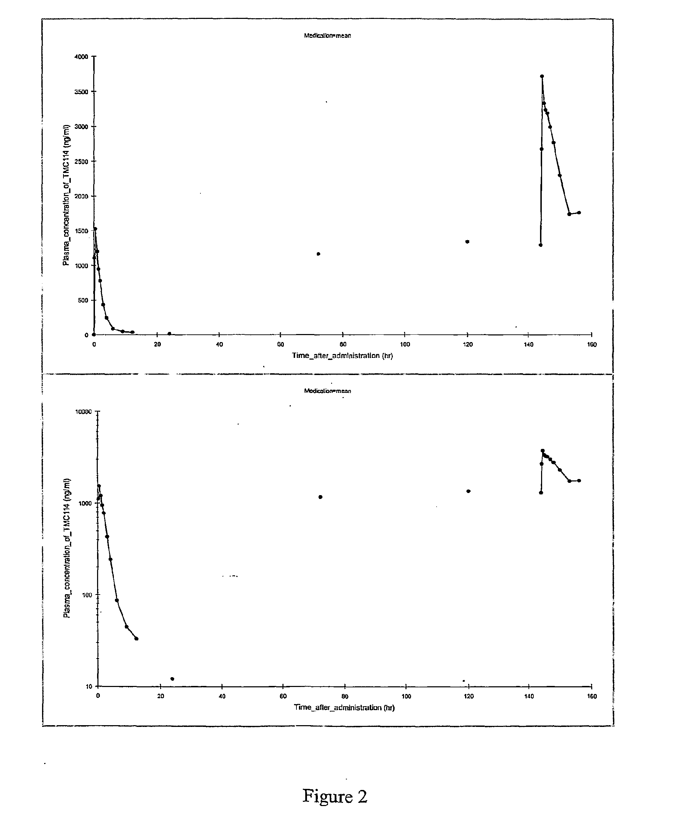

Figure 2: Represents a mean concentration-time profile of the first 7 days from a clinical trial with a combination of the compound of formula **(4)** with ritonavir, wherein the panel was subjected to oral administration of 400 mg the compound of formula **(4)** o.d. on day 1-14 and 100 mg ritonavir o.d. on day 2-16. The bottom figure is on a logarithmic scale.

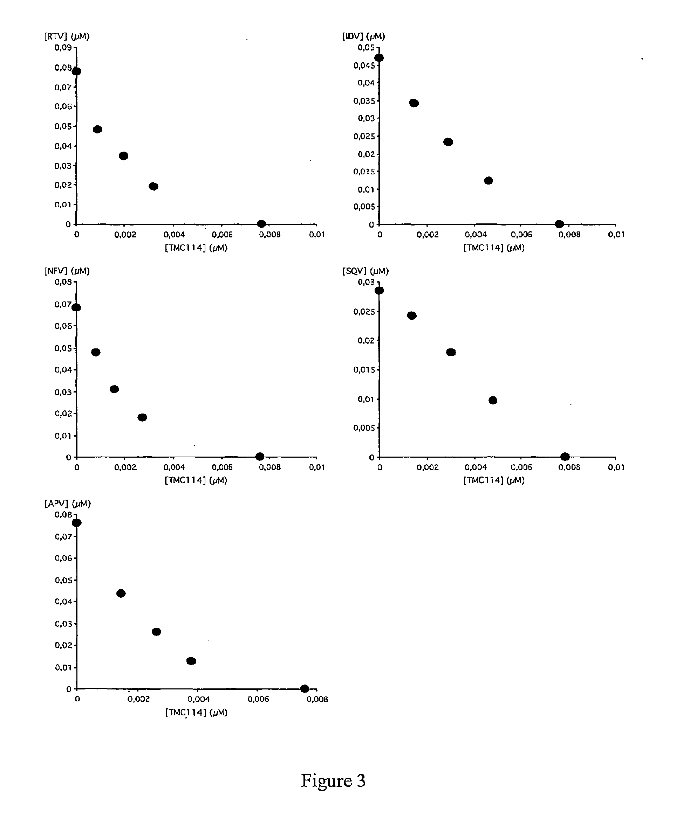

Figure 3: isobolograms for the combinations of the compound of formula **(4)** with HIV protease inhibitors. RTV: ritonavir; IDV: indinavir; NFV: nelfinavir; SQV: saquinavir; APV: amprenavir; TMC114: a compound of formula **(4).**

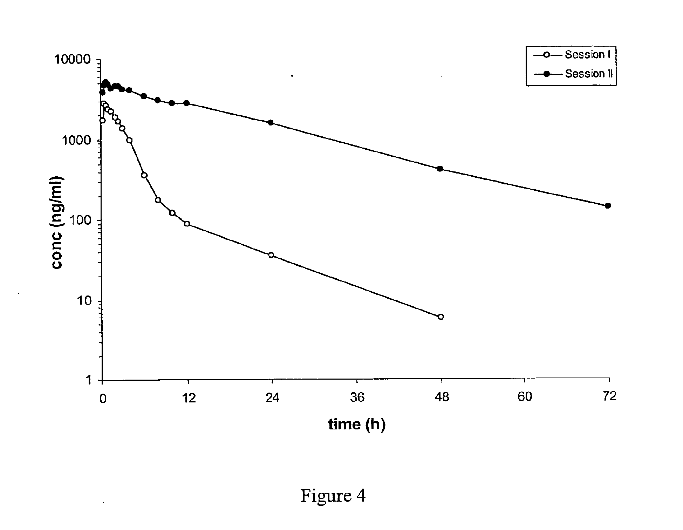

Figure 4: Mean plasma concentration-time profiles of a single 800 mg of the compound of formula **(4)** dose in the absence and presence of 'steady-state' concentrations of ritonavir (RTV) (600 mg b.i.d.) on a semi-logarithmic scale. (session 1, n=10 volunteers, the compound of formula **(4)** only; session 2, the compound of formula **(4)** + ritonavir, n=9 volunteers: 6 volunteers had their dose of RTV lowered to 400 mg b.i.d. or discontinued RTV intake from day 4).

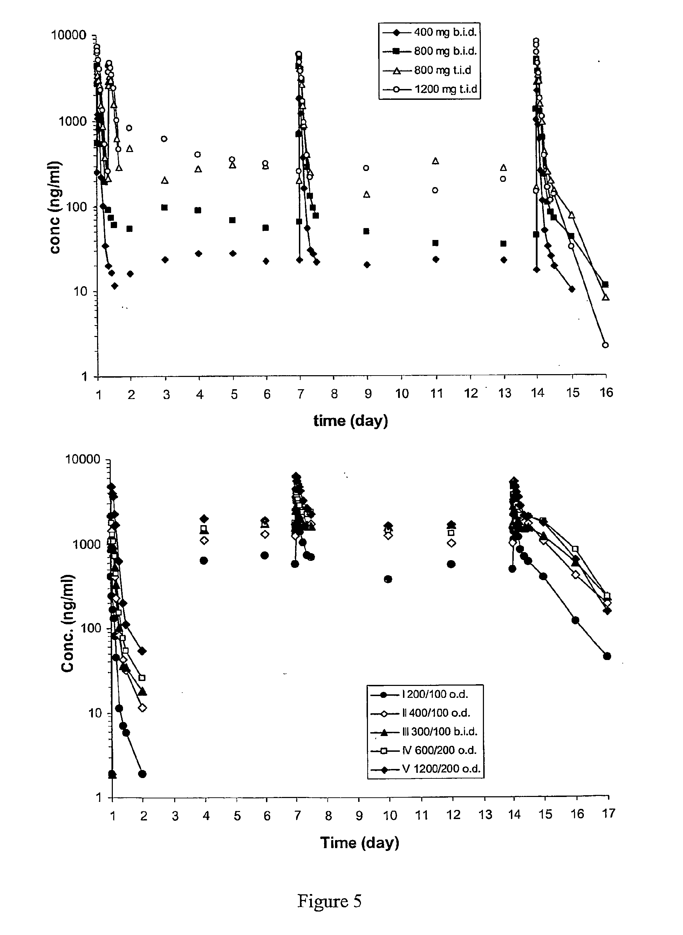

Figure 5 Top: Mean plasma concentration-time profiles of the compound of formula **(4)** at different dose levels on a semi-logarithmic scale (on day 1 and 7, n=6 per dose level, and on day 14, n=6 for 400 mg b.i.d., n=4 for 800 mg b.i.d., n=3 for 800 mg t.i.d. and n=2 for 1200 mg t.i.d.). Figure 5 bottom: Mean plasma concentration-time profiles of the compound of formula **(4)** at different dose levels in the presence of low doses of RTV on a semi-logarithmic scale (n=8 per panel). On day 1, a single dose of the compound of formula **(4)** was administered. From day 2 onwards, both the compound of formula **(4)** and RTV were administered. The regimens indicated in table consist of 200 mg of the compound of formula **(4)**/100 mg ritonavir; 400 mg of the compound of formula (**4**)/100 mg ritonavir; 300 mg of the compound of formula **(4)**/100 mg ritonavir; 600 mg of the compound of formula **(4)**/ 200 mg ritonavir; 1200 mg of the compound of formula **(4)**/ 200 mg ritonavir.

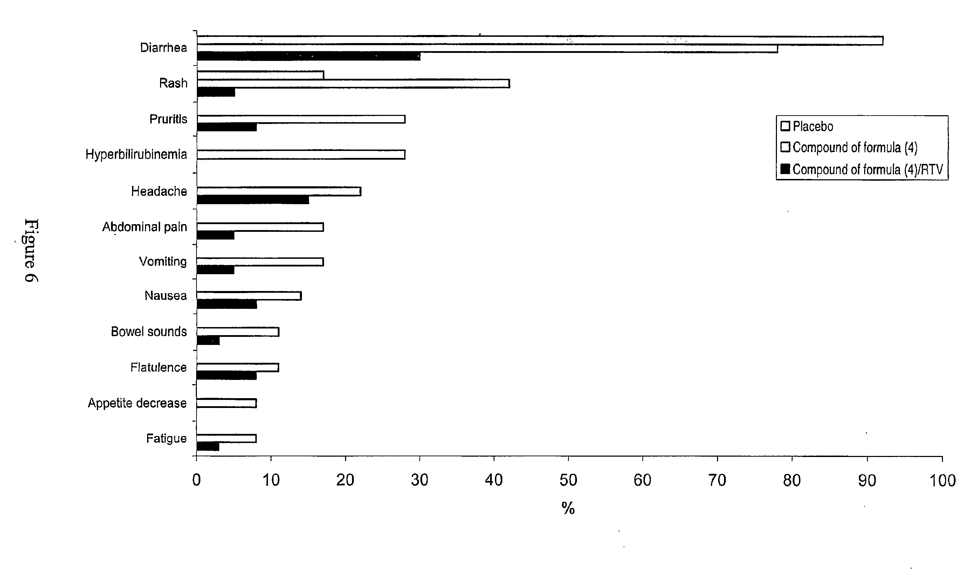

Figure 6: Adverse events that occurred in at least 2 individuals. The results are expressed as a percentage of the total group.
   Placebo: the group to which a placebo was administered.
   Compound of formula **(4):** The adverse events that occurred in the total population of individuals to whom the compound of formula **(4)** was administered.
   Compound of formula **(4)**/RTV: The adverse events that occurred in the total population of individuals to whom the compound of formula **(4)** was administered in combination with ritonavir.

In order that those skilled in the art will better understand the practice of the present invention, examples of the present invention are given below by way of illustration.

### Example 1. Influence of ritonavir on the pharmacokinetic variables of the compound of formula (4)

The pharmacokinetic variables for the compound of formula **(4)** where compared when the compound of formula **(4)** was administered alone to when the compound of formula **(4)** was co-administered to individuals to which ritonavir was given. The influence of ritonavir on the pharmacokinetics of a single dose of the compound of formula **(4)** is shown in Figure 4.

**Table I: Influence of ritonavir lowered to 400 mg b.i.d. or discontinued RTV intake from day 4.**

| Pharmacokinetic of the compound of formula (**4**) (tₘₐₓ: median (range); mean±SD) | Session I (the compound of formula (**4**) alone (n=12) | Session II (the compound of formula (**4**) with RTV) (n=9) |
|---|---|---|
| tₘₐₓ, h | 0.8 (0.3-2.5) | 1.0 (0.3-4.0) |
| Cₘₐₓ, ng/ml | 3306 ± 1487 | 6220 ± 2826 |
| AUC, ng.h/ml | 10713 ± 3126 | 98729 ± 38481 |
| t_{1/2}, h | 11.3 ± 4.62 | 12.2 ± 4.03 |

| | | |
|---|---|---|
| tₘₐₓ, h: time expressed in hours to obtain maximal concentration; Cmax, ng/ml:maximal concentration, expressed in ng/ml; AUC, ng.h/ml area under curve, expressed in ng x hours/ml; t _{1/2}, h: half life,expressed in hours | | |

### Example 2. Clinical testing of a combination of the compound of formula (4) with ritonavir

This experiment investigated the influence of low doses of ritonavir on the pharmacokinetics of the compound of formula **(4)** (n=8 per panel).

In panel A, 200 mg of the compound of formula **(4)** once daily (o.d.) was given in combination with 100 mg ritonavir o.d. On day 1 a single 200 mg dose the compound of formula **(4)** was given without ritonavir. The concentration decreased to about 3 ng/ml after 24 h (Figure 1). However, after combining 200 mg of the compound of formula **(4)** with 100 mg ritonavir o.d., the Cₘᵢₙ (minimum serum concentration) levels of the compound of formula **(4)** increased to a mean of 560 ng/ml (range 90-1300 ng/ml) (see table II). This means that addition of ritonavir caused a 200-fold increase in Cₘᵢₙ levels of the compound of formula **(4).**

As can be seen in the table below, Cₘᵢₙ levels at day 14 were comparable with the Cₘᵢₙ levels at day 7. At day 14, mean Cₘᵢₙ levels were 480 ng/ml, while Cₘᵢₙ levels at day 7 were 562 ng/ml. At both days, the interindividual variation was high, as can be seen in the wide range of Cₘᵢₙ levels. Both Cₘₐₓ (maximum serum concentration) and exposure levels were also comparable at both days.
In panel B, 400 mg the compound of formula **(4)** o.d. was given in combination with 100 mg ritonavir o.d. At this dose level, the mean Cₘᵢₙ level at day 7 was 1226 ng/ml. This means that by increasing the compound of formula **(4)** dose by 2, the Cₘᵢₙ levels were also increased by 2. Panel C has received 300 mg of the compound of formula **(4)** b.i.d. and 100 mg ritonavir b.i.d. for 14 days. Panel D has received 600 mg of the compound of formula **(4)** o.d. and 200 mg ritonavir o.d. for 14 days. Panel E has received 1200 mg of the compound of formula **(4)** o.d. and 200 mg ritonavir o.d. for 14 days. In comparison to panel D (600 mg of the compound of formula **(4)** o.d./200 mg ritonavir o.d.), Cₘᵢₙ levels of panel E were not increased. At day 7, mean Cₘᵢₙ levels were 1740 ng/ml for panel D and 1682 ng/ml for panel E. In both panels, Cₘᵢₙ levels were decreased at day 14. At day 14, mean Cₘᵢₙ levels were 1511 ng/ml for panel D and 1486 ng/ml for panel E.
In summary, co-administration of ritonavir led to much higher average and trough plasma concentrations of the compound of formula **(4)** at lower total daily dose levels of the compound of formula **(4).** Peak concentrations were lower or comparable. The safety profile of the compound of formula **(4)** in combination with low doses of ritonavir was good (cfr. Fig. 6). Unexpectedly, the combination of the compound of formula **(4)** together with ritonavir resulted in a reduced incidence of adverse effects. Unexpectedly, the combination has an improved safety and tolerability profile compared to therapy with the compound of formula **(4)** alone.
No maculopapular rash was observed for the volunteers in panels A to D. This was unexpected because the average and Cₘᵢₙ plasma concentrations of the compound of formula **(4)** were generally much higher than those after the compound of formula **(4)** was administered alone (In a study after 1200 mg of compound of formula **(4)** t.i.d. alone, there were 4 out of 6 subjects, who developed maculopapular rash). Cₘₐₓ levels were lower or comparable.
In panel E, there was one volunteer with a clear maculopapular rash. Furthermore, there were two other volunteers with itching of the body and/or redness of the skin. It is likely that a certain compound **(4)** metabolite causes the maculopapular rash. Inhibition of CYP3A4 metabolism will lead to lower levels of compound **(4)** metabolite and thus to a lower incidence of maculopapular rash. In panel E, there may be less inhibition due to the competition for the enzyme leading to more compound **(4)** metabolite formation. The advantage of the combination of RTV with the compound of formula **(4)** for therapy is further substantiated by the pharmacokinetic data in tables III toIV. C_{ss,av} means the average steady state concentration.

**Table II: Mean values and range of the Cₘᵢₙ, the Cₘₐₓ, the C_{ss,av} and the AUC₂₄ₕ of the compound of formula (4) with low doses of RTV at the different dose regimens (AUC = area under the curve i.e., total exposure of drug; Cₘₐₓ = maximum serum concentration, t.i.d. three times a day)**

| Pharmacokinetics of the compound of formula **(4)** (mean (range)) | Cₘᵢₙ (ng/ml) | Cₘₐₓ (ng/ml) | C_{ss,av} (ng/ml)^{*} | AUC₂₄ₕ (ng.h/ml)^{**} |
|---|---|---|---|---|
| Panel A (200 mg compound of formula **(4)** /100 mg RTV o.d.) | | | | |
| Day 7 (n = 7) | 562 (90-1290) | 1750 (1190-3630) | 857 (370-1739) | 20562 (8870-41725) |
| Day 14 (n = 7) | 480 (188-910) | 1569 (1090-2370) | 725 (374-1192) | 17409 (8971-28614) |

| Panel B (400 mg compound of formula **(4)**/100 mg RTV o.d.) | | | | |
|---|---|---|---|---|
| Day 7 (n = 8) | 1226 (551-1850) | 3540 (2440-5060) | 1851 (1157-2674) | 44414(27780-64178) |
| Day 14 (n = 8) | 981 (688-1710) | 3125 (2150-4650) | 1703 (1108-3385) | 40879(26585-81238) |

| Panel C (300 mg compound of formula **(4)**/100 mg RTV b.i.d.) | | | | |
|---|---|---|---|---|
| Day 7 (n = 8) | 1539 (832-2500) | 2893 (2310-3780) | 1892 (1095-2645) | 45408 (26270-63486) |
| Day 14(n=7) | 1650 (532-4350) | 2854(1910-5330) | 1771(970-4075) | 42500(23280-97800) |

| Panel D (600 mg compound of formula **(4)**/200 mg RTV o.d.) | | | | |
|---|---|---|---|---|
| Day 7 (n = 8) | 1740 (764-3290) | 4196 (2890-5820) | 2327 (1568-3036) | 55839 (37621-72865) |
| Day 14 (n = 8) | 1511 (817-2720) | 4628 (2790-5910) | 2188 (1345-3914) | 52505 (32282-93925) |

| Panel E (1200 mg compound of formula **(4)**/200 mg RTV o.d.) | | | | |
|---|---|---|---|---|
| Day 7 (n = 8) | 1682 (44-3090) | 6438 (3680-9400) | 2767 (908-4231) | 66399(21799-101534) |
| Day 14 (n = 7) | 1486 (203-2980) | 5453 (3520-7290) | 2460 (1122-3737) | 59045 (26925-89679) |

| | | | | |
|---|---|---|---|---|
| *C_{ss,av} the dosing interval (in hours) corresponds to the AUC for that dosing interval ** Extrapolated AUC₂₄ₕ (for b.i.d. 2*AUC₁₂ₕ) | | | | |

**Table III: Mean values and ranges of the Cₘᵢₙ, the Cₘₐₓ, the C_{ss,av} and the AUC₂₄ₕ for the different dose regimens. (AUC = area under the curve i.e., total exposure of drug; Cₘₐₓ = maximum serum concentration, t.i.d. three times a day)**

| Pharmacokinetics of the compound of formula (4) (mean (range)) | Cₘᵢₙ (ng/ml) | Cₘₐₓ (ng/ml) | C_{ss,av} (ng/ml)^{*} | AUC₂₄ₕ (ng.h/ml)^{**} |
|---|---|---|---|---|
| Panel F (400 mg of the compound of formula **(4)** b.i.d.) | | | | |
| Day 7 (n = 6) | 23 (5-45) | 2458 (1270-3540) | 321 (203-458) | 7702 (4864-10990) |
| Day 14 (n = 6) | 17 (6-30) | 2168 (1430-3270) | 270 (185-333) | 6477 (4438-7988) |

| Panel G (800 mg of the compound of formula **(4)** b.i.d.) | | | | |
|---|---|---|---|---|
| Day 7 (n = 6) | 64 (38-84) | 5493 (3800-6570) | 1033 (606-1414) | 24798 (14554-33938) |
| Day 14 (n =4) | 44 (32-52) | 5755 (3950-7240) | 951 (768-1103) | 23202 (18442-26474) |

| Panel H (800 mg of the compound of formula **(4)** t.i.d) | | | | |
|---|---|---|---|---|
| Day 7 (n = 6) | 197 (89-432) | 5227 (3910-6870) | 1463 (849-1876) | 35102 (20370-45024) |
| Day 14 (n = 3) | 161 (57-303) | 5143 (4880-5510) | 1506 (1253-1933) | 36131 (30075-46383) |

| Panel I (1200 mg of the compound of formula **(4)** t.i.d) | | | | |
|---|---|---|---|---|
| Day 7 (n = 6) | (125-504) | 6332 (3130-8980) | 1714 (966-2234) | 41121 (23175-53616) |
| Day 14 (n = 2) | 142 (78-206) | 8040 (7710-8370) | 2027 (1909-2144) | 48639 (45813-51465) |

| | | | | |
|---|---|---|---|---|
| C_{ss,av} time dosing interval corresponds to the AUC for that dosing interval** Extrapolated AUC₂₄ₕ (for b.i.d. 2*AUC₁₂ₕ, for t.i.d. 3*AUC₈ₕ) | | | | |

**Table IV: Mean values and range of Cₘᵢₙ, Cₘₐₓ, C_{ss,av} and AUC₂₄ₕ for the compound of formula (4) at different dosages with low doses of RTV**

| Pharmacokinetics of the compound of formula **(4)** (mean (range)) | Cₘᵢₙ (ng/ml) | Cₘₐₓ (ng/ml) | C_{ss,av} (ng/ml)^{*} | AUC₂₄ₕ (ng.h/ml)^{**} |
|---|---|---|---|---|
| Panel J (300 mg compound of formula (**4**)/100 mg RTV b.i.d.) | | | | |
| Day 14 (n = 12) | 1175 (684-1890) | 4440 (2490-10200) | 2129 (45-3384) | 51092(27476-81226) |

| Panel K (600 mg compound of formula (**4**)/100 mg RTV b.i.d.) | | | | |
|---|---|---|---|---|
| Day 14 (n = 12) | 1819 (612-5270) | 5738(2760-9160) | 2915 (1049-6404) | 69953 (25174-153696) |

| Panel L (900 mg compound of formula (**4**)/100 mg RTV o.d.) | | | | |
|---|---|---|---|---|
| Day 14 (n=9) | 1438 (468-2140) | 6549 (4710-7870) | 2651(1833-3018) | 63611 (43985-72430) |

| | | | | |
|---|---|---|---|---|
| *C_{ss,av} the dosing interval (in hours) corresponds to the AUC for that dosing interval ** Extrapolated AUC₂₄ₕ (for b.i.d. 2*AUC₁₂ₕ) | | | | |

### Example 3 : Synergy of combinations of the compound of formula (4) and other HIV protease inhibitors (this example comprises comparative data)

The activity of combinations of the compound of formula **(4)** with the current anti-HIV drugs at three different molar ratios was determined in HIV-1/LAI infected MT4 cells. The results were analyzed according to the isobologram method described by Chou and Talalay (1984)

The results are presented as the mean of three separate experiments. The combination index (CI) for each combination was determined. A CI value between 0.8 and 1.2 reflects additive inhibition of the combined compounds, a value below 0.8 indicates a synergy between the two molecules, whereas a value greater than 1.2 is indicative of antagonism.

The compound of formula **(4)** exhibited no antagonism with any of the tested drugs. It showed additive inhibition with indinavir (CI: 0.87-0.92), lopinavir (CI: 0.85-0.95) and saquinavir (0.94-1.0), at all molar ratios, and it showed synergy with amprenavir (CI: 0.65-0.77), nelfinavir (0.61-0.80) and ritonavir (0.66-0.81), at all molar ratios.

These results are also illustrated in Figure 3 where the isobolograms for the combinations of the compound of formula **(4)** with HIV protease inhibitors respectively are plotted. Whereas a straight line represents additive inhibition by two inhibitors, a curve towards the origin of the axes indicates synergy. The latter is observed for combinations with amprenavir, nelfinavir and ritonavir.

## Claims

1. Combination comprising (a) an HIV protease inhibitor of formula **(4)** 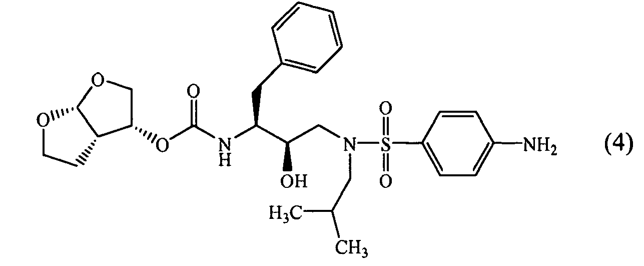 or a pharmaceutically acceptable salt thereof and (b) ritonavir or a pharmaceutically acceptable salt thereof.

2. Combination according to claim 1 wherein the components comprised in said combination are to be administered together or separately.

3. Combination according to claim 1 or 2 wherein the weight ratio of the HIV protease inhibitor of formula **(4)** to ritonavir is in the range of from 40:1 1 to 1:15.

4. Combination according to any one of claims 1 to 3 wherein the weight ratio of the HIV protease inhibitor of formula **(4)** to ritonavir is in the range of from 30:1 to 1:15.

5. Combination according to any one of claims 1 to 4 wherein the weight ratio of the HIV protease inhibitor of formula **(4)** to ritonavir is in the range of from 10: 1 to 1:10.

6. Combination according to any one of claims 1 to 5 wherein the weight ratio of the HIV protease inhibitor of formula **(4)** to ritonavir is in the range of from 8:1 to 1:8.

7. Combination according to any one of claims 1 to 6 wherein the weight ratio of the HIV protease inhibitor of formula **(4)** to ritonavir is in the range of from 6:1 to 1:6.

8. Combination according to any one of claims 1 to 7 wherein the amount by weight of HIV protease inhibitor of formula **(4)** is equal to or greater than that of ritonavir.

9. Combination according to claim 8 wherein the weight ratio of HIV protease inhibitor of formula **(4)** to ritonavir is in the range of from 1: 1 to 15: 1.

10. Combination according to claim 8 or 9 wherein the weight ratio of HIV protease inhibitor of formula **(4)** to ritonavir is in the range of from 1: 1 to 8: 1.

11. Combination according to any one of claims 8 to 10 wherein the weight ratio of HIV protease inhibitor of formula **(4)** to ritonavir is in the range of from 1: 1 to 6: 1.

12. Combination according to claim 1 or 2, wherein the components comprised in said combination are to be co-administered twice a day, wherein the amount of the compound of formula (4) per dose is from 50 to 1500 mg and the amount of ritonavir per dose is from 50 to 1500 mg of ritonavir.

13. Combination according to claim 12, wherein the amount of the compound of formula (4) per dose is from 100 to 1000 mg and the amount of ritonavir per dose is from 100 to 800 mg.

14. Combination according to claim 12, wherein the amount of the compound of formula (4) per dose is from 150 to 800 mg and the amount of ritonavir per dose is from 100 to 600 mg.

15. Combination according to claim 12, wherein the amount of the compound of formula (4) per dose is from 200 to 600 mg and the amount of ritonavir per dose is from 20 to 300 mg.

16. Combination according to claim 1 or 2 wherein the components comprised in said combination are to be co-administered twice a day, wherein the amount of the compound of formula (4) per dose is 600 mg and the amount of ritonavir per dose is 100 mg of ritonavir.

17. Combination according to claim 1 or 2, wherein the components comprised in said combination are to be co-administered once a day, wherein the amount of the compound of formula (4) per dose is from 50 to 1500 mg and the amount of ritonavir per dose is from 50 to 1500 mg of ritonavir.

18. Combination according to claim 17, wherein the amount of the compound of formula (4) per dose is from 100 to 1000 mg and the amount of ritonavir per dose is from 100 to 800 mg.

19. Combination according to claim 17, wherein the amount of the compound of formula (4) per dose is from 150 to 800 mg and the amount of ritonavir per dose is from 100 to 600 mg.

20. Combination according to claim 17, wherein the amount of the compound of formula (4) per dose is from 200 to 600 mg and the amount of ritonavir per dose is from 20 to 200 mg.

21. Pharmaceutical composition comprising a therapeutic amount of a combination according to any one of claims 1 to 20 and a pharmaceutically acceptable excipient.

22. Kit containing (a) a pharmaceutical composition comprising a therapeutic amount of an HIV protease inhibitor of formula **(4)** 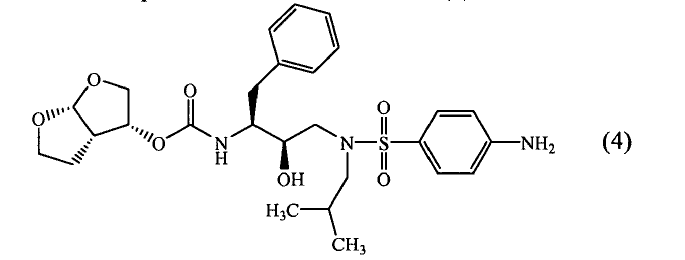 or a pharmaceutically acceptable salt thereof, and (b) ritonavir or a pharmaceutically acceptable salt thereof, as a combined preparation for simultaneous, separate or sequential use in HIV therapy.

23. A combination according to any one of claims 1 to 20 for use as a medicament.

24. Use of a combination according to any one of claims 1 to 20 in the manufacture of a medicament for treating, preventing or combating infection or disease associated with retrovirus infection in a mammal.

25. Use of a combination according to any one of claims 1 to 20 in the manufacture of a medicament for treating or combating infection or disease associated with retrovirus infection in a mammal.

26. Use of a combination according to any one of claims 1 to 20 in the manufacture of a medicament for inhibiting a protease of a retrovirus in a mammal infected with said retrovirus.

27. Use of a combination according to any of claims 1 to 20 in the manufacture of a medicament for inhibiting retroviral replication.

28. Use according to any one of claims 24 to 27 wherein the retrovirus is a human immunodeficiency virus (HIV).

29. Use according to any of claims 24 to 28, wherein the retrovirus is a multidrug-resistant retrovirus.

30. Use of a combination according to any of claims 1 to 20 for improving the pharmacokinetics of a compound of formula **(4)** relative to the pharmacokinetics when a compound of formula **(4)** is administered alone, in the manufacture of a medicament for the inhibition of viral proteases.

31. Use of a combination according to any of claims 1 to 20 in the manufacture of a medicament for the treatment or prevention of HIV or HIV related conditions comprising AIDS in a human, **characterized in that** said combination is useful for improving the pharmacokinetic variables of a compound of formula **(4)** relative to the pharmacokinetic variables when a compound of formula **(4)** is administered alone.

32. Use of a combination according to claim 30, wherein the amount of ritonavir or a pharmaceutically acceptable salt thereof is sufficient for increasing at least one of the pharmacokinetic variables selected from Cₘᵢₙ, Cₘₐₓ, AUC at 12 hours, relative to the pharmacokinetic variables when a compound of formula **(4)** is administered alone.

33. Use of a combination according to claim 30, wherein the amount of ritonavir or a pharmaceutically acceptable salt thereof is sufficient for increasing at least one of the pharmacokinetic variables of a compound of formula **(4)** selected from Cₘᵢₙ, Cₘₐₓ, C_{ss,av}, AUC at 12 hours, or AUC at 24 hours, relative to said at least one pharmacokinetic variable when a compound of formula **(4)** is administered alone.

## Patentansprüche

1. Kombination, umfassend (a) einen HIV-Protease-Inhibitor der Formel **(4)** 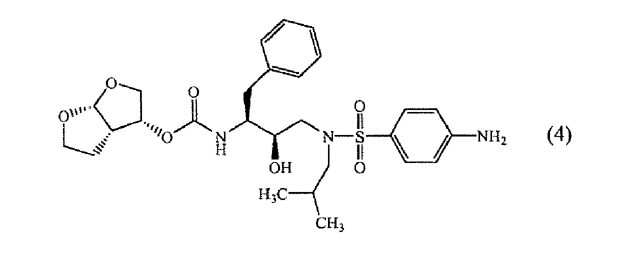 oder ein pharmazeutisch unbedenkliches Salz davon und (b) Ritonavir oder ein pharmazeutisch unbedenkliches Salz davon.

2. Kombination nach Anspruch 1, wobei die in der Kombination enthaltenen Komponenten zusammen oder getrennt zu verabreichen sind.

3. Kombination nach Anspruch 1 oder 2, wobei das Gewichtsverhältnis des HIV-Protease-Inhibitors der Formel **(4)** zu Ritonavir im Bereich von 40:1 bis 1:15 liegt.

4. Kombination nach einem der Ansprüche 1 bis 3, wobei das Gewichtsverhältnis des HIV-Protease-Inhibitors der Formel **(4)** zu Ritonavir im Bereich von 30:1 bis 1:15 liegt.

5. Kombination nach einem der Ansprüche 1 bis 4, wobei das Gewichtsverhältnis des HIV-Protease-Inhibitors der Formel **(4)** zu Ritonavir im Bereich von 10:1 bis 1:10 liegt.

6. Kombination nach einem der Ansprüche 1 bis 5, wobei das Gewichtsverhältnis des HIV-Protease-Inhibitors der Formel **(4)** zu Ritonavir im Bereich von 8:1 bis 1:8 liegt.

7. Kombination nach einem der Ansprüche 1 bis 6, wobei das Gewichtsverhältnis des HIV-Protease-Inhibitors der Formel **(4)** zu Ritonavir im Bereich von 6:1 bis 1:6 liegt.

8. Kombination nach einem der Ansprüche 1 bis 7, wobei die Gewichtsmenge an HIV-Protease-Inhibitor der Formel **(4)** gleich der oder größer als die von Ritonavir ist.

9. Kombination nach Anspruch 8, wobei das Gewichtsverhältnis von HIV-Protease-Inhibitor der Formel **(4)** zu Ritonavir im Bereich von 1:1 bis 15:1 liegt.

10. Kombination nach Anspruch 8 oder 9, wobei das Gewichtsverhältnis von HIV-Protease-Inhibitor der Formel **(4)** zu Ritonavir im Bereich von 1:1 bis 8:1 liegt.

11. Kombination nach einem der Ansprüche 8 bis 10, wobei das Gewichtsverhältnis von HIV-Protease-Inhibitor der Formel **(4)** zu Ritonavir im Bereich von 1:1 bis 6:1 liegt.

12. Kombination nach Anspruch 1 oder 2, wobei die in der Kombination enthaltenen Komponenten zweimal täglich zusammen zu verabreichen sind, wobei die Menge der Verbindung der Formel (4) pro Dosis 50 bis 1500 mg und die Menge an Ritonavir pro Dosis 50 bis 1500 mg Ritonavir beträgt.

13. Kombination nach Anspruch 12, wobei die Menge der Verbindung der Formel (4) pro Dosis 100 bis 1000 mg und die Menge an Ritonavir pro Dosis 100 bis 800 mg beträgt.

14. Kombination nach Anspruch 12, wobei die Menge der Verbindung der Formel (4) pro Dosis 150 bis 800 mg und die Menge an Ritonavir pro Dosis 100 bis 600 mg beträgt.

15. Kombination nach Anspruch 12, wobei die Menge der Verbindung der Formel (4) pro Dosis 200 bis 600 mg und die Menge an Ritonavir pro Dosis 20 bis 300 mg beträgt.

16. Kombination nach Anspruch 1 oder 2, wobei die in der Kombination enthaltenen Komponenten zweimal täglich zusammen zu verabreichen sind, wobei die Menge der Verbindung der Formel (4) pro Dosis 600 mg und die Menge an Ritonavir pro Dosis 100 mg Ritonavir beträgt.

17. Kombination nach Anspruch 1 oder 2, wobei die in der Kombination enthaltenen Komponenten einmal täglich zusammen zu verabreichen sind, wobei die Menge der Verbindung der Formel (4) pro Dosis 50 bis 1500 mg und die Menge an Ritonavir pro Dosis 50 bis 1500 mg Ritonavir beträgt.

18. Kombination nach Anspruch 17, wobei die Menge der Verbindung der Formel (4) pro Dosis 100 bis 1000 mg und die Menge an Ritonavir pro Dosis 100 bis 800 mg beträgt.

19. Kombination nach Anspruch 17, wobei die Menge der Verbindung der Formel (4) pro Dosis 150 bis 800 mg und die Menge an Ritonavir pro Dosis 100 bis 600 mg beträgt.

20. Kombination nach Anspruch 17, wobei die Menge der Verbindung der Formel (4) pro Dosis 200 bis 600 mg und die Menge an Ritonavir pro Dosis 20 bis 200 mg beträgt.

21. Pharmazeutische Zusammensetzung, umfassend eine therapeutische Menge einer Kombination nach einem der Ansprüche 1 bis 20 sowie einen pharmazeutisch unbedenklichen Hilfsstoff.

22. Kit, enthaltend (a) eine pharmazeutische Zusammensetzung, umfassend eine therapeutische Menge eines HIV-Protease-Inhibitors der Formel **(4)** 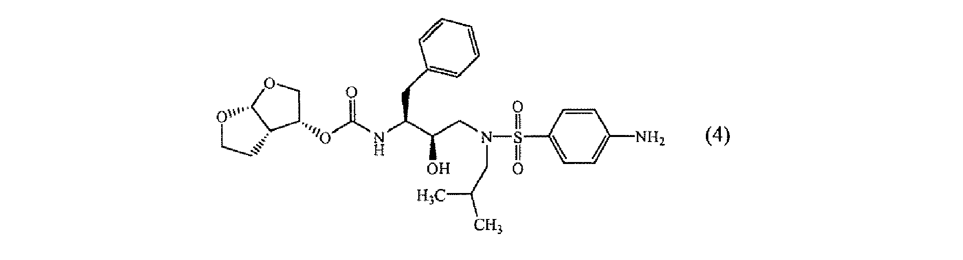 oder ein pharmazeutisch unbedenkliches Salz davon und (b) Ritonavir oder ein pharmazeutisch unbedenkliches Salz davon, als Kombinationspräparat für die gleichzeitige, getrennte oder aufeinanderfolgende Verwendung bei der HIV-Therapie.

23. Kombination nach einem der Ansprüche 1 bis 20 zur Verwendung als Arzneimittel.

24. Verwendung einer Kombination nach einem der Ansprüche 1 bis 20 bei der Herstellung eines Arzneimittels zur Behandlung, Vorbeugung oder Bekämpfung von mit Retrovirusinfektion assoziierter Infektion oder Krankheit in einem Säuger.

25. Verwendung einer Kombination nach einem der Ansprüche 1 bis 20 bei der Herstellung eines Arzneimittels zur Behandlung oder Bekämpfung von mit Retrovirusinfektion assoziierter Infektion oder Krankheit in einem Säuger.

26. Verwendung einer Kombination nach einem der Ansprüche 1 bis 20 bei der Herstellung eines Arzneimittels zur Hemmung einer Protease eines Retrovirus in einem mit dem Retrovirus infizierten Säuger.

27. Verwendung einer Kombination nach einem der Ansprüche 1 bis 20 bei der Herstellung eines Arzneimittels zur Hemmung der retroviralen Replikation.

28. Verwendung nach einem der Ansprüche 24 bis 27, wobei es sich bei dem Retrovirus um ein menschliches Immunschwächevirus (HIV) handelt.

29. Verwendung nach einem der Ansprüche 24 bis 28, wobei es sich bei dem Retrovirus um ein gegen mehrere Arzneistoffe resistentes Retrovirus handelt.

30. Verwendung einer Kombination nach einem der Ansprüche 1 bis 20 zur Verbesserung der Pharmakokinetik einer Verbindung der Formel **(4)** relativ zu der Pharmakokinetik bei alleiniger Verabreichung einer Verbindung der Formel **(4)** bei der Herstellung eines Arzneimittels zur Hemmung viraler Proteasen.

31. Verwendung einer Kombination nach einem der Ansprüche 1 bis 20 bei der Herstellung eines Arzneimittels zur Behandlung oder Vorbeugung von HIV oder mit HIV verbundenen, AIDS umfassenden Leiden beim Menschen, **dadurch gekennzeichnet, dass** sich die Kombination zur Verbesserung der pharmakokinetischen Variablen einer Verbindung der Formel **(4)** relativ zu den pharmakokinetischen Variablen bei alleiniger Verabreichung einer Verbindung der Formel **(4)** eignet.

32. Verwendung einer Kombination nach Anspruch 30, wobei die Menge an Ritonavir oder einem pharmazeutisch unbedenklichen Salz davon zur Erhöhung wenigstens einer der aus Cₘᵢₙ, Cₘₐₓ, AUC zum Zeitpunkt 12 Stunden ausgewählten pharmakokinetischen Variablen relativ zu den pharmakokinetischen Variablen bei alleiniger Verabreichung einer Verbindung der Formel **(4)** ausreicht.

33. Verwendung einer Kombination nach Anspruch 30, wobei die Menge an Ritonavir oder einem pharmazeutisch unbedenklichen Salz davon zur Erhöhung wenigstens einer der aus Cₘᵢₙ, Cₘₐₓ, C_{ss,av}, AUC zum Zeitpunkt 12 Stunden oder AUC zum Zeitpunkt 24 Stunden ausgewählten pharmakokinetischen Variablen einer Verbindung der Formel **(4)** relativ zu der wenigstens einen pharmakokinetischen Variablen bei alleiniger Verabreichung einer Verbindung der Formel **(4)** ausreicht.

## Revendications

1. Combinaison comprenant (a) un inhibiteur de protéase du VIH de formule (4) 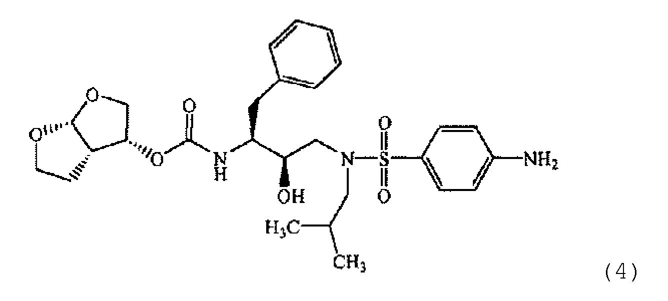 ou un sel pharmaceutiquement acceptable de celui-ci et (b) du ritonavir ou un sel pharmaceutiquement acceptable de celui-ci.

2. Combinaison selon la revendication 1 **caractérisé en ce que** les composants compris dans ladite combinaison sont destinés à être administrés conjointement ou séparément.

3. Combinaison selon la revendication 1 ou 2 **caractérisée en ce que** le rapport en poids de l'inhibiteur de protéase du VIH de formule (4) au ritonavir est dans la plage de 40:1 à 1:15.

4. Combinaison selon l'une quelconque des revendications 1 à 3 **caractérisée en ce que** le rapport en poids de l'inhibiteur de protéase du VIH de formule (4) au ritonavir est dans la plage de 30:1 à 1:15.

5. Combinaison selon l'une quelconque des revendications 1 à 4 **caractérisée en ce que** le rapport en poids de l'inhibiteur de protéase du VIH de formule (4) au ritonavir est dans la plage de 10:1 à 1:10.

6. Combinaison selon l'une quelconque des revendications 1 à 5 **caractérisée en ce que** le rapport en poids de l'inhibiteur de protéase du VIH de formule (4) au ritonavir est dans la plage de 8:1 à 1:8.

7. Combinaison selon l'une quelconque des revendications 1 à 6 **caractérisée en ce que** le rapport en poids de l'inhibiteur de protéase du VIH de formule (4) au ritonavir est dans la plage de 6:1 à 1:6.

8. Combinaison selon l'une quelconque des revendications 1 à 7 **caractérisée en ce que** la quantité en poids d'inhibiteur de protéase du VIH de formule (4) est égale ou supérieure à celle du ritonavir.

9. Combinaison selon la revendication 8 **caractérisée en ce que** le rapport en poids d'inhibiteur de protéase du VIH de formule (4) au ritonavir est dans la plage de 1:1 à 15:1.

10. Combinaison selon la revendication 8 ou 9
**caractérisée en ce que** le rapport en poids d'inhibiteur de protéase du VIH de formule (4) au ritonavir est dans la plage de 1:1 à 8:1.

11. Combinaison selon l'une quelconque des revendications 8 à 10 **caractérisée en ce que** le rapport en poids d'inhibiteur de protéase du VIH de formule (4) au ritonavir est dans la plage de 1:1 à 6:1.

12. Combinaison selon la revendication 1 ou 2, **caractérisée en ce que** les composants compris dans ladite combinaison sont destinés à être co-administrés deux fois par jour, où la quantité du composé de formule (4) par dose est de 50 à 1500 mg et la quantité de ritonavir par dose est de 50 à 1500 mg of ritonavir.

13. Combinaison selon la revendication 12, **caractérisée en ce que** la quantité du composé de formule (4) par dose est de 100 à 1000 mg et la quantité de ritonavir par dose est de 100 à 800 mg.

14. Combinaison selon la revendication 12, **caractérisée en ce que** la quantité du composé de formule (4) par dose est de 150 à 800 mg et la quantité de ritonavir par dose est de 100 à 600 mg.

15. Combinaison selon la revendication 12, **caractérisée en ce que** la quantité du composé de formule (4) par dose est de 200 à 600 mg et la quantité de ritonavir par dose est de 20 à 300 mg.

16. Combinaison selon la revendication 1 ou 2 **caractérisée en ce que** les composants compris dans ladite combinaison sont destinés à être co-administrés deux fois par jour, où la quantité du composé de formule (4) par dose est de 600 mg et la quantité de ritonavir par dose est de 100 mg de ritonavir.

17. Combinaison selon la revendication 1 ou 2, **caractérisée en ce que** les composants compris dans ladite combinaison sont destinés à être co-administrés deux fois par jour, où la quantité du composé de formule (4) par dose est de 50 à 1500 mg et la quantité de ritonavir par dose est de 50 à 1500 mg de ritonavir.

18. Combinaison selon la revendication 17, **caractérisée en ce que** la quantité du composé de formule (4) par dose est de 100 à 1000 mg et la quantité de ritonavir par dose est de 100 à 800 mg.

19. Combinaison selon la revendication 17, **caractérisée en ce que** la quantité du composé de formule (4) par dose est de 150 à 800 mg et la quantité de ritonavir par dose est de 100 à 600 mg.

20. Combinaison selon la revendication 17, **caractérisée en ce que** la quantité du composé de formule (4) par dose est de 200 à 600 mg et la quantité de ritonavir par dose est de 20 à 200 mg.

21. Composition pharmaceutique comprenant une quantité thérapeutique d'une combinaison selon l'une quelconque des revendications 1 à 20 et un excipient pharmaceutiquement acceptable.

22. Kit contenant (a) une composition pharmaceutique comprenant une quantité thérapeutique d'un inhibiteur de protéase du VIH de formule (4) 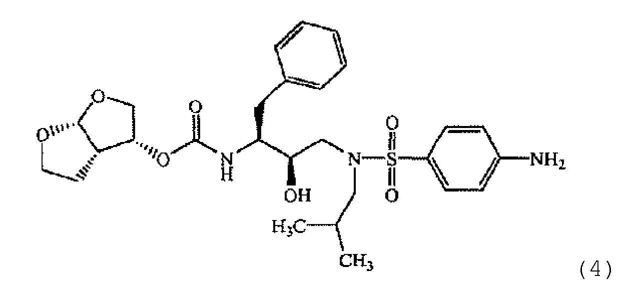 ou un sel pharmaceutiquement acceptable de celui-ci et (b) du ritonavir ou un sel pharmaceutiquement acceptable de celui-ci, sous la forme d'une préparation combinée pour utilisation simultanée, séparée ou séquentielle dans la thérapie du VIH.

23. Combinaison selon l'une quelconque des revendications 1 à 20 pour utilisation en tant que médicament.

24. Utilisation d'une combinaison selon l'une quelconque des revendications 1 à 20 dans la fabrication d'un médicament pour traiter, prévenir ou combattre une infection ou une maladie associée à une infection par un rétrovirus chez un mammifère.

25. Utilisation d'une combinaison selon l'une quelconque des revendications 1 à 20 dans la fabrication d'un médicament pour traiter ou combattre une infection ou une maladie associée à une infection par un rétrovirus chez un mammifère.

26. Utilisation d'une combinaison selon l'une quelconque des revendications 1 à 20 dans la fabrication d'un médicament pour inhiber une protéase d'un rétrovirus chez un mammifère infecté par ledit rétrovirus.

27. Utilisation d'une combinaison selon l'une quelconque des revendications 1 à 20 dans la fabrication d'un médicament pour inhiber la réplication rétrovirale.

28. Utilisation selon l'une quelconque des revendications 24 à 27 **caractérisée en ce que** le rétrovirus est un virus d'immunodéficience humaine (VIH).

29. Utilisation selon l'une quelconque des revendications 24 à 28, **caractérisée en ce que** le rétrovirus est un rétrovirus multirésistant aux médicaments.

30. Utilisation d'une combinaison selon l'une quelconque des revendications 1 à 20 pour améliorer la pharmacocinétique d'un composé de formule (4) par rapport à la pharmacocinétique lorsqu'un composé de formule (4) est administré seul, dans la fabrication d'un médicament pour l'inhibition de protéases virales.

31. Utilisation d'une combinaison selon l'une quelconque des revendications 1 à 20 dans la fabrication d'un médicament pour le traitement ou la prévention du VIH ou de pathologies associées au VIH comprenant le SIDA chez un humain, **caractérisée en ce que** ladite combinaison est utile pour améliorer les variables pharmacocinétiques d'un composé de formule (4) par rapport aux variables pharmacocinétiques lorsqu'un composé de formule (4) est administré seul.

32. Utilisation d'une combinaison selon la revendication 30, **caractérisée en ce que** la quantité de ritonavir ou un sel pharmaceutiquement acceptable de celui-ci est suffisante pour augmenter au moins une des variables pharmacocinétiques choisies parmi Cₘᵢₙ, Cₘₐₓ, ASC à 12 heures, par rapport aux variables pharmacocinétiques lorsqu'un composé de formule (4) est administré seul.

33. Utilisation d'une combinaison selon la revendication 30, **caractérisée en ce que** la quantité de ritonavir ou un sel pharmaceutiquement acceptable de celui-ci est suffisante pour augmenter au moins une des variables pharmacocinétiques d'un composé de formule (4) choisies parmi Cₘᵢₙ, Cₘₐₓ, C_{ss,av}, ASC à 12 heures, ou ASC à 24 heures, par rapport à ladite au moins une variable pharmacocinétique lorsqu'un composé de formule (4) est administré seul.
